# FASCICULE DE BREVET EUROPEEN

(11) **EP 1 031 343 B1**
(45) Date de publication et mention de la délivrance du brevet: **07.11.2001**
(21) Numéro de dépôt: 00400149.1
(22) Date de dépôt: 20.01.2000
(51) Int. Cl.: A61K 7/135

(54) **Composition aqueuse de décoloration de fibres kératiniques prête à l'emploi comprenant l'association d'un solvant hydrosoluble et d'un polymère amphiphile non ionique ou anionique comportant au moins une chaîne grasse**
Gebrauchsfertige wässrige Zusammensetzung zum Bleichen von Keratinfasern, die ein wasserlösliches Lösungsmittel in Kombination mit einem nichtionischen oder anionischen amphiphilen Polymer enthält, das mindestens eine Fettkette aufweist
Ready-to-use aqueous composition for bleaching of keratinic fibre, containing a combination of a water soluble solvent and a non-ionic amphiphilic or anionic amphiphilic polymer with at least one fatty chain

(30) Priorité: 29.01.1999 FR 9901055
(43) Date de publication de la demande: 30.08.2000
(73) Titulaire: L'OREAL, 75008 Paris (FR)
(72) Inventeur: Legrand, Frédéric, 92100 Boulogne-Billancourt (FR); Millequant, Jean, 94100 Saint-Maur (FR)
(74) Mandataire: Casalonga, Axel

(56) Documents cités:
- EP-A- 0 827 738
- EP-A- 0 882 444
- WO-A-97/07776
- US-A- 3 940 351

## Description

La présente invention concerne des compositions aqueuses prêtes à l'emploi pour la décoloration de fibres kératiniques comprenant l'association d'au moins un solvant hydrosoluble et d'au moins un polymère amphiphile non ionique et /ou anionique comportant au moins une chaîne grasse, un procédé de décoloration de fibres kératiniques utilisant ces compositions, ainsi qu'un kit d'emballage contenant une telle composition.

Pour décolorer les cheveux, on peut utiliser des compositions aqueuses résultant du mélange, au moment de l'emploi, d'une composition aqueuse de peroxyde d'hydrogène, d'une composition aqueuse contenant un agent alcalin et au moins un solvant hydrosoluble et d'une poudre contenant un réactif peroxygéné tel que les persulfates, perborates ou percarbonates d'ammonium ou de métaux alcalins.

Dans le domaine de la décoloration capillaire, on recherche généralement des compositions décolorantes suffisamment épaisses pour permettre une application précise sur certaines zones de la chevelure, et qui ne risquent pas de couler sur le visage ou en dehors des zones que l'on se propose de décolorer.

L'effet épaississant ou gélifiant est classiquement obtenu avec des épaississants traditionnels tels que les dérivés de cellulose, les dérivés d'amidon, les alginates, les silices épaississantes ou des mélanges de tensioactifs judicieusement choisis.

La demande de brevet européen EP-A-0 882 444 divulgue une composition aqueuse pour la décoloration des cheveux comprenant une combinaison de huit composants essentiels dont un composant épaississant macromoléculaire qui est une combinaison d'un polymère d'acide acrylique et d'un polymère d'origine naturelle choisi parmi les celluloses, les alginates et les polysaccharides.

La demande de brevet européen EP-A-0 827 738 divulgue des compositions de teinture d'oxydation pour fibres kératiniques comprenant dans un milieu approprié pour la teinture, au moins un précurseur de colorant d'oxydation et, éventuellement, un ou plusieurs coupleurs et qui sont caractérisées par le fait quelles comprennent en outre un polymère amphiphile anionique à fonctions acide carboxylique et à fonctions ester d'alkyle en C₁₀₋₃₀.

Lorsqu'on utilise ces épaississants traditionnels, on constate cependant une importante diminution de la viscosité de la composition décolorante finale au cours du temps.

Il existe donc un besoin d'un système épaississant capable d'assurer le maintien d'une viscosité élevée pendant la durée nécessaire pour obtenir la décoloration souhaitée, généralement comprise entre dix minutes et une heure.

La demanderesse a fait la découverte surprenante qu'il était possible d'améliorer considérablement le maintien de la viscosité au cours du temps des compositions de décoloration décrites ci-dessus, en associant au système initial, un polymère amphiphile non ionique et/ou anionique comportant au moins une chaîne grasse.

On a également constaté qu'un tel système épaississant permettait des dilutions, avec des compositions aqueuses de peroxyde d'hydrogène, nettement plus importantes que les systèmes épaississants connus.

La présente invention a par conséquent pour objet une composition aqueuse prête à l'emploi de décoloration de fibres kératiniques, en particulier humaines, comprenant, dans un milieu approprié pour la décoloration, au moins un agent alcalin, au moins un solvant hydrosoluble, du peroxyde d'hydrogène, au moins un sel peroxygéné, et en outre au moins un polymère amphiphile non ionique et/ou anionique comportant au moins une chaîne grasse.

Elle a en outre pour objet un procédé de décoloration des fibres kératiniques utilisant la composition de décoloration aqueuse décrite ci-dessus, ainsi qu'un kit d'emballage contenant une telle composition.

D'autres objets apparaîtront à la lecture de la description et des exemples qui suivront.

Les polymères amphiphiles non ioniques comportant au moins une chaîne grasse utilisables selon la présente invention englobent par exemple :
- les celluloses ou hydroxyalkylcelluloses modifiées par des groupements comportant au moins une chaîne grasse telle qu'un groupe alkyle, arylalkyle ou alkylaryle contenant un groupe alkyle en C₈₋₂₂ comme les produits NATROSOL® PLUS GRADE 330 CS de la société AQUALON, BERMOCOLL® EHM 100 de la société BEROL NOBEL, ou POLYSURF® 67 de la société HERCULES, ou modifiées par des groupes alkylphénol polyalcoxylés comme le produit AMERCELL POLYMER® HM-1500 de la société AMERCHOL;
- les hydroxypropylguars modifiés par des groupements comportant au moins une chaîne grasse en C₈₋₂₂ comme les produits ESAFLOR® HM 22 (chaîne alkyle en C₂₂) de la société LAMBERTI, MIRACARE® XC95-3 (chaîne alkyle en C₁₄) et RE205-1 (chaîne alkyle en C₂₀) de la société RHONE POULENC;
- les polyuréthannes comportant au moins une chaîne grasse de type alkyle ou alcényle en C₈₋₃₀ comme le SER-AD® FX 1100 de la société SERVO DELBEN;
- le copolymère SMDI (Saturated Methylene Diphenyl Diisocyanate) polyéthylèneglycol(s) avec terminaison décyle ;
- le copolymère SMDI Saturated Methylene Diphenyl Diisocyanate)polyéthylèneglycol(s) avec terminaison alkyle (méthyle/C18), associé à une matrice maltodextrine ;
- le diuréthane HMDI (Hexa Methylene Di Isocyanate) d'alcools E10-C18 oxyéthylénés (66 OE) et oxypropylénés (14 OP) commercialisé sous l'appelation ELFACOS® T 212 par la société AKZO ;
- les copolymères de vinylpyrrolidone et de monomères hydrophobes à chaîne grasse comme les produits ANTARON® V216 ou GANEX® V216 (poly(vinylpyrrolidone/hexadécène)), ANTARON® V220 ou GANEX® V220 (poly(vinylpyrrolidone/eicosène)) de la société I.S.P.;
- les copolymères de (méth)acrylates d'alkyle en C₁₋₆ et de monomères amhiphiles comportant au moins une chaîne grasse;
- les copolymères de (méth)acrylates hydrophiles et de monomères hydrophobes comportant au moins une chaîne grasse, par exemple un poly(méthacrylate de polyéthylèneglycol/méthacrylate de lauryle).

On préfère en particulier les polyuréthannes comportant au moins une chaîne grasse de type alkyle en C₁₀₋₂₀ et les hydroxyéthylcelluloses modifiées par des groupements comportant au moins une chaîne alkyle en C₈₋₂₂.

Les polymères amphiphiles anioniques comportant au moins une chaîne grasse utilisés selon la présente invention sont des copolymères, réticulés ou non réticulés, comprenant
- des motifs *hydrophiles* dérivés d'un ou de plusieurs monomères à insaturation éthylénique portant une fonction d'acide carboxylique libre, et
- des motifs *hydrophobes* dérivés d'un ou de plusieurs monomères à insaturation éthylénique portant une chaîne latérale hydrophobe, et éventuellement
- des motifs de réticulation dérivés d'un ou de plusieurs monomères polyinsaturés.

Le ou les monomères à insaturation éthylénique portant une fonction d'acide carboxylique sont choisis parmi l'acide éthacrylique, L'acide méthacrylique et l'acide acrylique, de préférence parmi l'acide méthacrylique, l'acide acrylique et des mélanges de ceux-ci.

Le ou les monomères à insaturation éthylénique portant une chaîne latérale hydrophobe peuvent être (i) des esters d'acides carboxyliques insaturés et d'alcools gras, ou (ii) des éthers d'allyle et d'alcools gras.

(i) Les esters d'acides carboxyliques insaturés et d'alcools gras sont choisis par exemple parmi les éthacrylates, méthacrylates et/ou acrylates d'alkyle en C₁₀₋₃₀, de préférence en C₁₂₋₂₂.

Ils englobent par exemple l'acrylate de lauryle, l'acrylate de stéaryle, l'acrylate de décyle, l'acrylate d'isodécyle, l'acrylate de dodécyle ainsi que les méthacrylates correspondants, à savoir le méthacrylate de lauryle, le méthacrylate de stéaryle, le méthacrylate de décyle, le méthacrylate d'isodécyle et le méthacrylate de dodécyle.

(ii) Les éthers allyliques d'alcools gras formant les motifs hydrophobes des polymères amphiphiles anioniques de la présente invention correspondent à la formule

(I) CH₂=CR'CH₂-O-Bₙ-R

dans laquelle
R' représente un atome d'hydrogène ou un groupe méthyle,
B représente un groupe éthylèneoxy,
n est un nombre entier valant entre 0 et 100,
R représente un groupe hydrocarboné choisi parmi les restes alkyle, arylalkyle, aryle, alkylaryle ou cycloalkyle comportant de 8 à 30 atomes de carbone, de préférence de 10 à 24 atomes de carbone, et plus particulièrement de 12 à 18 atomes de carbone.

Un motif de formule (I) préféré selon la présente invention est un motif dans lequel R' désigne un atome d'hydrogène, n est égal à 10 et R représente un radical stéaryle (C₁₈).

Ledit monomère réticulant est un composé comportant au moins deux doubles liaisons polymérisables non conjuguées l'une avec l'autre. On peut citer à titre d'exemple le phtalate de diallyle, le (méth)acrylate d'allyle, le divinylbenzène, le diméthacrylate de (poly)éthylèneglycol, le méthylène-bis-acrylamide, le polyallylsucrose ou le polyallylpentaérythritol.

Des polymères amphiphiles anioniques du type décrit ci-dessus sont décrits par exemple dans les brevets US-3 915 921 et US-4 509 949 (copolymères d'acide (éth/méth)acrylique et de (éth/méth)acrylates d'alkyle en C₁₀₋₃₀), ou dans le brevet EP-0 216 479 B2 (copolymères d'acide (éth/méth)acrylique et d'éthers allyliques d'alcools gras).

On peut citer à titre d'exemples de polymères préférés :
- les polymères réticulés d'acide acrylique et d'acrylate d'alkyle en C₁₀₋₃₀, tels que les polymères commercialisés sous les dénominations PEMULEN® TR1, PEMULEN® TR2 et CARBOPOL® 1382 par la société GOODRICH,
- le polymère réticulé d'acide acrylique et de méthacrylate d'alkyle en C ₁₀₋₃₀, tel que le CARBOPOL® ETD 2020 commercialisé par la société GOODRICH,
- le terpolymère acide méthacrylique/acrylate d'éthyle/méthacrylate de stéaryle oxyéthyléné (55/35/10) ;
- le terpolymère acide (méth)acrylique/acrylate d'éthyle/méthacrylate de béhényle oxyéthyléné (25 OE), et
- le terpolymère acide méthacrylique/acrylate d'éthyle/stéareth10 allyléther réticulé.

Ces polymères amphiphiles non ioniques et/ou anioniques comportant au moins une chaîne grasse sont utilisés à raison de 0,01 à 10 % en poids, de préférence à raison de 0,1 à 5 % en poids par rapport à la composition décolorante aqueuse.

Comme indiqué ci-dessus, la composition décolorante aqueuse contient également au moins un agent alcalin, au moins un sel peroxygéné, au moins un solvant hydrosoluble et du peroxyde d'hydrogène.

Ledit agent alcalin est choisi parmi l'ammoniaque et les amines organiques telles que la mono-, di- ou triéthanolamine, le 2-amino-2-méthyl-1-propanol, le diaminopropane, les hydroxyalkylamines et les éthylènediamines oxyéthylénées et/ou oxypropylénées.

Les sels peroxygénés sont choisis parmi les persulfates, les percarbonates et les perborates d'ammonium ou de métaux alcalins.

On utilise de préférence les persulfates et parmi ceux-ci principalement les persulfates de sodium et de potassium.

Les compositions de l'invention contiennent de 2 % à 20 % en poids et de préférence de 4 à 15 % en poids de sel peroxygéné.

Au sens de la présente invention, on entend par "solvants hydrosolubles" des solvants solubles à plus de 5 % en poids dans l'eau à 25 °C.

Les solvants hydrosolubles de l'invention sont choisis de préférence parmi les monoalcools aliphatiques linéaires ou ramifiés en C₁₋₆, les polyols aliphatiques linéaires ou ramifiés en C₃₋₂₀, les éthers de polyols dont les mono- ou di-éthers aliphatiques en C₁₋₆ de polyols en C₂₋₉ et les éthers aromatiques en C₆₋₉ de polyols en C₂₋₉.

Les compositions de l'invention contiennent de 0,1 à 10 % en poids, de préférence de 0,5 à 8 % en poids, de solvant hydrosoluble.

Elles contiennent également entre 0,5 et 10 % en poids, de préférence entre 1 % et 8 % en poids, de peroxyde d'hydrogène.

Les compositions de décoloration aqueuses selon la présente invention peuvent également contenir toutes sortes d'adjuvants de décoloration susceptibles de faciliter la manipulation et l'application, d'améliorer la conservation ou l'efficacité des compositions et d'améliorer les propriétés cosmétiques des cheveux traités.

Ces adjuvants sont par exemple des agents de contrôle du dégagement d'oxygène tels que le carbonate de magnésium et la magnésie, des agents tensioactifs anioniques, non ioniques, cationiques, amphotères ou zwitterioniques et leurs mélanges, des huiles minérales ou végétales, des cires, des liants, des charges minérales telles que la silice et l'argile, des opacifiants tels que l'oxyde de titane, des colorants, des séquestrants, des parfums et des polymères.

Les compositions de l'invention peuvent contenir en particulier au moins un polymère hydrosoluble épaississant, naturel ou synthétique et/ou au moins un polymère substantif cationique et/ou amphotère.

Bien entendu, l'homme de métier veillera à choisir ce (ou ces) éventuel(s) composé(s) supplémentaire(s) et sa ou (leur) quantité de manière à ce que les propriétés avantageuses attachées intrinsèquement à la composition de décoloration conforme à l'invention ne soient pas, ou substantiellement pas, altérées par la (ou les) adjonction(s) envisagée(s).

Les compositions de l'invention comprennent de préférence au moins un agent tensio-actif.

Les tensioactifs convenant à la mise en oeuvre de la présente invention sont notamment les suivants

### (i) Tensio-actif(s) anionique(s) :

A titre d'exemple de tensio-actifs anioniques utilisables, seuls ou mélanges, dans le cadre de la présente invention, on peut citer notamment les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl(C₆-C₂₄) sulfosuccinates, les alkyl(C₆-C₂₄) éthersulfosuccinates, les alkyl(C₆-C₂₄) amidesulfosuccinates ; les alkyl(C₆-C₂₄) sulfoacétates ; les acyl(C₆-C₂₄) sarcosinates et les acyl(C₆-C₂₄) glutamates. On peut également utiliser les esters d'alkyl(C₆-C₂₄)polyglycosides carboxyliques tels que les alkylglucoside citrates, les alkylpolyglycoside tartrate et les alkylpolyglycoside sulfosuccinates., les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

### (ii) Tensio-actif(s) non ionique(s) :

Les agents tensio-actifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi les alcools, les alpha-diols, les alkylphénols ou les acides gras polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensio-actifs non-ioniques rentrant particulièrement bien. dans le cadre de la présente invention.

### (iii) Tensio-actif(s) amphotère(s) ou zwitterionique(s) :

Les agents tensio-actifs amphotères ou zwitterioniques, dont la nature ne revet pas dans le cadre de la présente invention de caractère critique, peuvent être notamment des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (parexemplecarboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₁-CONHCH₂CH₂-N(R₂)(R₃)(CH₂COO-)

dans laquelle : R₂ désigne un radical alkyle d'un acide R₁-COOH présent dans l'huile de coprah hydrolysée, un radical heptyle, nonyle ou undécyle, R₂ désigne un groupement bêta-hydroxyéthyle et R₃ un groupement carboxyméthyle ;
et

R₁'-CONHCH₂CH₂-N(B)(C)

dans laquelle :
B représente -CH₂CH₂OX',
C représente -(CH₂)_{z} -Y', avec z = 1 ou 2,
X' désigne le groupement -CH₂CH₂-COOH ou un atome d'hydrogène
Y' désigne -COOH ou le radical -CH₂-CHOH-SO₃H
R₁' désigne un radical alkyle d'un acide R-COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5^{ème} édition, 1993, sous les dénominations Disodium Coco-ampho-diacetate, Disodium Lauroampho-diacetate, Disodium Capryl-ampho-diacetate, Disodium Capryloampho-diacetate, Disodium Coco-amphodipropionate, Disodium Lauroampho-dipropionate, Disodium Caprylampho-dipropionate, Disodium Capryloampho-dipropionate, Lauroampho-dipropionic acid, Coco-ampho-dipropionic acid.

### (iv) Tensio-actifs cationiques :

Parmi les tensio-actifs cationiques on peut citer en particulier : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

Les quantités d'agents tensioactifs présents dans la composition selon l'invention peuvent représenter de 0,01 à 40% et de préférence de 0,1 à 30 % du poids total de la composition.

Les polymères substantifs cationiques utilisables conformément à la présente invention peuvent être choisis parmi tous ceux déjà connus en soi comme améliorant les propriétés cosmétiques des cheveux, à savoir notamment ceux décrits dans les demandes de brevet EP-A-337354 et EP-A-557203 et dans les brevets français FR-2 270 846, 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire.

Ce sont des produits connus. Ils sont notamment décrits dans les brevets français n° 2 505 348 ou 2 542 997. Parmi lesdits polymères, on peut citer :
(1) les homopolymères ou copolymères dérivés d'esters ou d'amides acryliques ou méthacryliques et comportant au moins un des motifs de formules (II), (III), (IV) ou (V) suivantes: 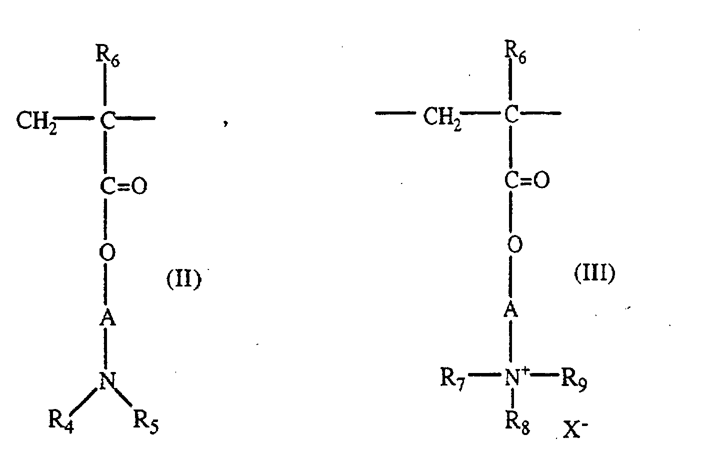 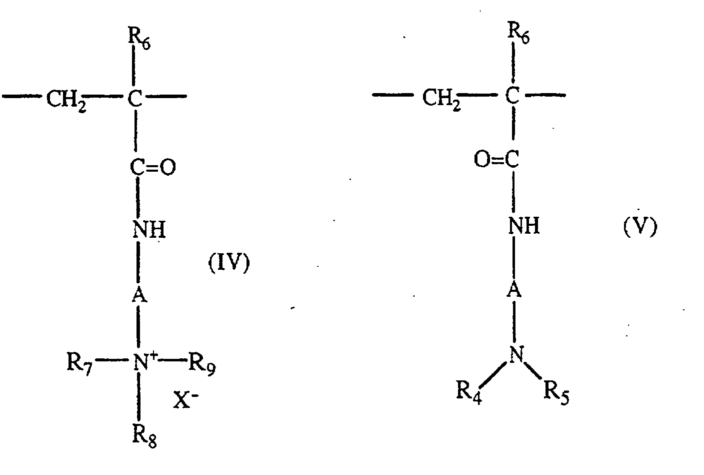 dans lesquelles:
   R₆, identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₇, R₈, R₉, identiques ou différents, représentent un groupe alkyle ayant de 1 à 18 atomes de carbone ou un radical benzyle et de préférence un groupe alkyle ayant de 1 à 6 atomes de carbone;
   R₄ et R₅, identiques ou différents, représentent hydrogène ou un groupe alkyle ayant de 1 à 6 atomes de carbone et de préférence méthyle ou éthyle;
   X désigne un anion dérivé d'un acide minéral ou organique tel qu'un anion méthosulfate ou un halogénure tel que chlorure ou bromure.

   Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques.
   Ainsi, parmi ces polymères de la famille (1), on peut citer :
   - les copolymères d'acrylamide et de méthacrylate diméthylaminoéthyle quaternisé au sulfate de diméthyle ou avec un halogénure de diméthyle,
   - les copolymères d'acrylamide et de chlorure de méthacryloyloxyéthyltriméthylammonium décrits par exemple dans la demande de brevet EP-A-080976,
   - le copolymère d'acrylamide et de méthosulfate de méthacryloyloxy-éthyltriméthyl-ammonium,
   - les copolymères vinylpyrrolidone/acrylate ou méthacrylate de dialkylaminoalkyle quaternisés ou non. Ces polymères sont décrits en détail dans les brevets français 2.077.143 et 2.393.573,
   - les terpolymères méthacrylate de diméthylaminoéthyle/vinylcaprolactame/vinylpyrrolidone,
   - les copolymère vinylpyrrolidone/méthacrylamidopropyldimethylamine,
   - et les copolymères vinylpyrrolidone/méthacrylamide de diméthyl-aminopropyle quaternisés.
(2) Les dérivés d'éthers de cellulose comportant des groupements ammonium quaternaire décrits dans le brevet français 1 492 597. Ces polymères sont également définis dans le dictionnaire CTFA comme des ammonium quaternaires d'hydroxyéthylcellulose ayant réagi avec un époxyde substitué par un groupement triméthylammonium.
(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkylcelluloses, comme les hydroxyméthyl-, hydroxyéthyl- ou hydroxypropylcelluloses greffées notamment avec un sel de méthacryloyléthyltriméthylammonium, de méthacrylmidopropyltriméthylammonium, de diméthyldiallylmmonium.
(4) Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyltriméthylammonium.
(5) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
(6) Les polyaminoamides solubles dans l'eau, préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épihalogénhydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 .
(7) Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-dialcoylaminohydroxyalcoyldialcoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine® F, F4 ou F8" par la société Sandoz.
(8) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire du polyalkylène polyamine à l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
(9) Les cyclopolymères d'alkyl diallyl amine ou de dialkyldiallylammonium tels que les homopolymères ou copolymères comportant comme constituant principal de la chaîne des motifs répondant aux formules (VI) ou (VII) : 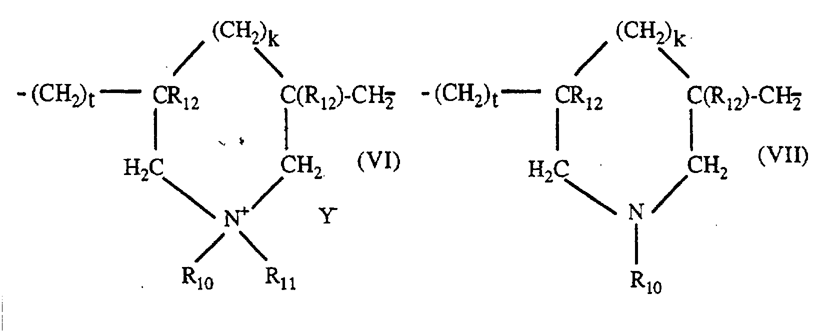 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (C₁-C₄), ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
(10) Le polymère de diammonium quaternaire contenant des motifs récurrents répondant à la formule : 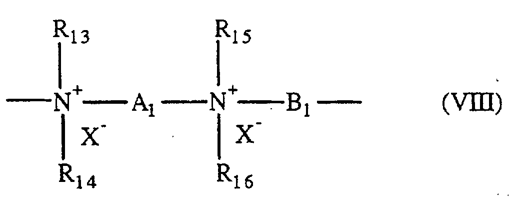 dans laquelle R₁₃, R₁₄, R₁₅ et R₁₆, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote ou bien R₁₃, R₁₄, R₁₅ et R₁₆ représentent un radical alkyle en C₁-C₆ linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire ;
   A₁ et B₁ représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A₁, R₁₃ et R₁₅ peuvent former avec les deux atomes d'azote auxquels ils sont rattachés un cycle pipérazinique ; en outre si A₁ désigne un radical alkylène ou hydroxyalkylène linéaire ou ramifié, saturé ou insaturé, B₁ peut également désigner un groupement

      -(CH₂)ₙ-CO-D-OC-(CH₂)ₙ-

      dans lequel D désigne :
      a) un reste de glycol de formule :
         -O-Z-O-, où Z désigne un radical hydrocarboné linéaire ou ramifié ou un groupement répondant à l'une des formules suivantes :

         -(CH₂-CH₂-O)ₓ-CH₂-CH₂-

         -[CH₂-CH(CH₃)-O]_{y}-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréylène de formule : -NH-CO-NH- ;

      De préférence, X⁻ est un anion tel que le chlorure ou le bromure.
      Ces polymères ont une masse moléculaire moyenne en nombre généralement comprise entre 1000 et 100000.
      Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
      On peut utiliser plus particulièrement les polymères qui sont constitués de motifs récurrents répondant à la formule (IX) suivante: 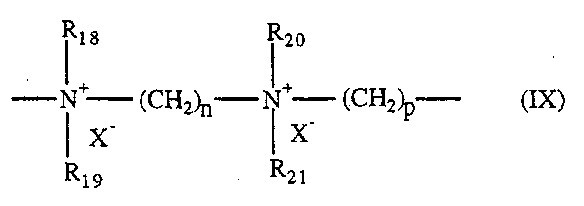 dans laquelle R₁₈, R₁₉, R₂₀ et R₂₁, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.
   (11) Les polymères de polyammonium quaternaires constitués de motifs de formule (X): 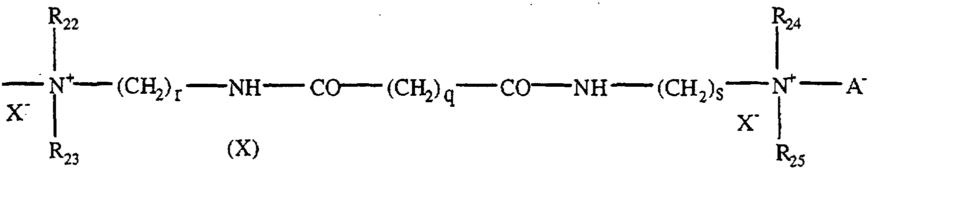 formule dans laquelle
      R₂₂, R₂₃, R₂₄ et R₂₅, identiques ou différents, représentent un atome d'hydrogène ou un radical méthyle, éthyle, propyle, β-hydroxyéthyle, β-hydroxypropyle ou -CH₂CH₂(OCH₂CH₂)ₚOH, où p est égal à 0 ou à un nombre entier compris entre 1 et 6, sous réserve que R₂₂, R₂₃, R₂₄ et R₂₅ ne représentent pas simultanément un atome d'hydrogène,
      r et s, identiques ou différents, sont des nombres entiers compris entre 1 et 6,
      q est égal à 0 ou à un nombre entier compris entre 1 et 34,
      X désigne un atome d'halogène,
      A désigne un radical d'un dihalogénure ou représente de préférence -CH₂-CH₂-O-CH₂-CH₂-.

      De tels composés sont notamment décrits dans la demande de brevet EP-A-122 324.
   (12) Les polymères quaternaires de vinylpyrrolidone et de vinylimidazole.
   (13) Les polyamines comme le produit référencé sous le nom de POLYETHYLENEGLYCOL (15) TALLOW POLYAMINE dans le dictionnaire CTFA.
   (14) Les polymères réticulés de sels de méthacryloyl-oxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homopolymérisation ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyltriméthylammonium (20/80 en poids) sous forme de dispersion contenant 50 % en poids dudit copolymère dans de l'huile minérale. Cette dispersion est commercialisée sous le nom de "SALCARE® SC 92 " par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de SALCARE® SC 95 et SALCARE® SC 96 par la Société ALLIED COLLOIDS.

D'autres polymères substantifs cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Les polymères substantifs amphotères utilisables conformément à la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwitterioniques de carboxybétaïnes ou de sulfobétaïnes;

K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères filmogènes amphotères répondant à la définition donnée ci-dessus plus particulièrement préférés sont choisis parmi les polymères suivants :
(1) Les polymères résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique tel que plus particulièrement les méthacrylates et -acrylates de dialkylaminoalkyle, les dialkylaminoalkylméthacrylamide et -acrylamide. De tels composés sont décrits dans le brevet américain n° 3 836 537. On peut également citer le copolymère acrylate de sodium/chlorure d'acrylamidopropyltriméthylammonium.
   Le composé vinylique peut être également un sel de dialkyldiallylammonium tel que le chlorure de diéthyldiallylammonium.
(2) Les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou de diéthyle.

   Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléïque, fumarique ainsi que les monoesters d'alkyle ayant 1 à 4 atomes de carbone des acides ou des anhydrides maléïque ou fumarique.
   Les comonomères basiques préférés sont des méthacrylates d'aminoéthyle, de butylaminoéthyle, de N,N'-diméthylaminoéthyle, de N-tertio-butylaminoéthyle.
   On utilise particulièrement les copolymères dont la dénomination CTFA (4^{ème} édition, 1991) est Octylacrylamide/acrylates/butylaminoethylmethacrylate copolymer.
(3) Les polyaminoamides réticulés et alcoylés partiellement ou totalement dérivant de polyaminoamides de formule générale :

   ⁅CO―R₂₆―CO―Z⁆ (XI)

   dans laquelle R₂₆ représente un radical divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical 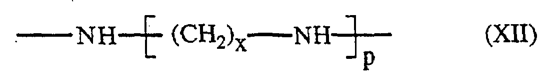 où x=2 et p=2 ou 3, ou bien x=3 et p=2,
      ce radical dérivant de la diéthylène triamine, de la triéthylène tétraamine ou de la dipropylène triamine;
   b) dans les proportions de 0 à 40 moles % le radical (XII) ci-dessus, dans lequel x=2 et p=1 et qui dérive de l'éthylènediamine, ou le radical dérivant de la pipérazine : 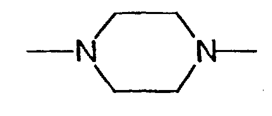
   c) dans les proportions de 0 à 20 moles % le radical

      -NH-(CH₂)₆-NH-

      dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.

   Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
   Les alcanesultones utilisées dans l'alcoylation sont de préférence la propanesultone ou la butanesultone, les sels des agents d'alcoylation sont de préférence les sels de sodium ou de potassium.
(4) Les polymères comportant des motifs zwitterioniques de formule : 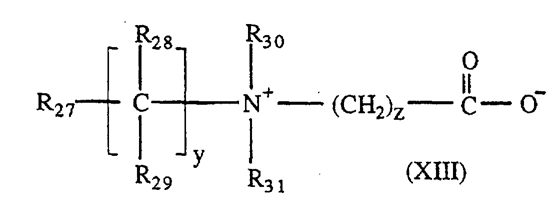 dans laquelle R₂₇ désigne un groupement insaturé polymérisable tel qu'un groupement acrylate, méthacrylate, acrylamide ou méthacrylamide, y et z représentent un nombre entier de 1 à 3, R₂₈ et R₂₉ représentent un atome d'hydrogène, méthyle, éthyle ou propyle, R₃₀ et R₃₁ représentent un atome d'hydrogène ou un radical alkyle de telle façon que la somme des atomes de carbone dans R₃₀ et R₃₁ ne dépasse pas 10.
   Les polymères comprenant de telles unités peuvent également comporter des motifs dérivés de monomères non zwitterioniques tels que l'acrylate ou le méthacrylate de diméthyl- ou diéthylaminoéthyle ou des acrylates ou méthacrylates d'alkyle, des acrylamides ou méthacrylamides ou l'acétate de vinyle.
   A titre d'exemple, on peut citer le copolymère de méthacrylate de méthyle/diméthylcarboxy-méthyl-ammonioéthyl-méthacrylate de méthyle.
(5) les polymères dérivés du chitosane comportant des motifs monomères répondant aux formules (XIV), (XV), (XVI) suivantes : 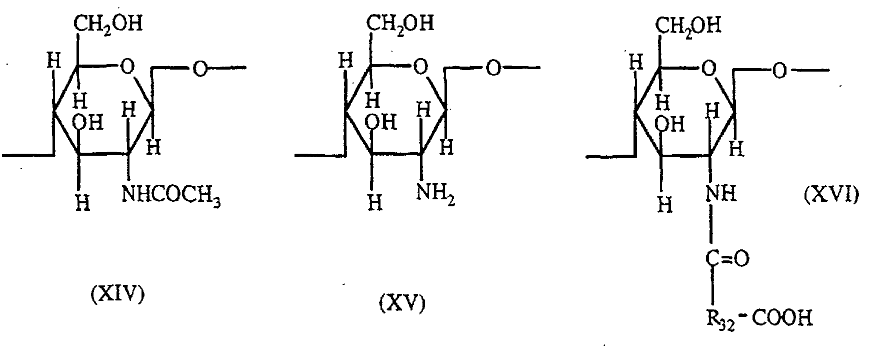 le motif XIV étant présent dans des proportions comprises entre 0 et 30%, le motif XV dans des proportions comprises entre 5 et 50% et le motif XVI dans des proportions comprises entre 30 et 90%, étant entendu que dans ce motif F, R₂₉ représente un radical de formule : 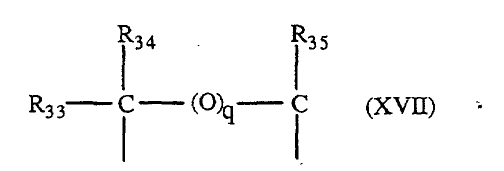 dans laquelle
   si q=0, R₃₃, R₃₄ et R₃₅, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcolylthio, sulfonique, un reste alcoylthio dont le groupe alcoyle porte un reste amino, l'un au moins des radicaux R₃₃, R₃₄ et R₃₅ étant dans ce cas un atome d'hydrogène ;
   ou si q=1, R₃₃, R₃₄ et R₃₅ représentent chacun un atome d'hydrogène, ainsi que les sels formés par ces composés avec des bases ou des acides.
(6) Les polymères dérivés de la N-carboxyalkylation du chitosane comme le N-carboxyméthyl chitosane ou le N-carboxybutylchitosane.
(7) Les polymères répondant à la formule générale (XVIII) tels que ceux décrits par exemple dans le brevet français 1 400 366 : 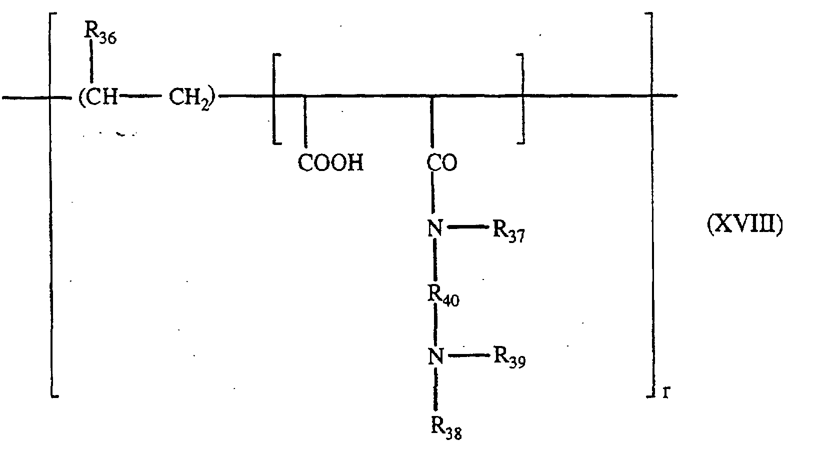 dans laquelle R₃₆ représente un atome d'hydrogène, un radical CH₃O, CH₃CH₂O, phényle, R₃₇ désigne l'hydrogène ou un radical alkyle inférieur tel que éthyle, R₃₈ désigne l'hydrogène ou un radical alkyle inférieur tel que méthyle, éthyle, R₃₉ désigne un radical alkyle inférieur tel que méthyle, éthyle ou un radical répondant à la formule :
   - R₄₀-N(R₃₈)₂, R₄₀ représentant un groupement -CH₂-CH₂-, - CH₂-CH₂-CH₂-, -CH₂-CH(CH₃)-, et R₃₈ ayant les significations mentionnées ci-dessus, ainsi que les homologues supérieurs de ces radicaux et contenant jusqu'à 6 atomes de carbone.
(8) Des polymères amphotères du type -D-X-D-X- choisis parmi:
   a) les polymères obtenus par action de l'acide chloracétique ou le chloracétate de sodium sur les composés comportant au moins un motif de formule

      -D-X-D-X-D- (XIX)

      où D désigne un radical 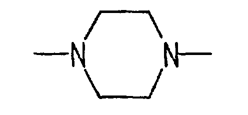 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
   b) les polymères de formule:

      -D-X-D-X- (XX)

      où D désigne un radical 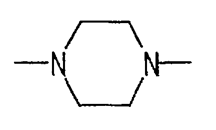 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) Les copolymères alkyl(C₁-C₅)-vinyléther/anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Parmi les polymères substantifs cationiques ou amphotères utilisables selon l'invention, on préfère notamment :
- l'homopolymère de chlorure de diméthyldiallylammonium vendu sous la dénomination MERQUAT 100 DRY par la société MERCK ;
- les copolymères de chlorure de diméthyldiallylammonium et d'acrylamide vendus sous la dénomination MERQUAT 2200 par la société CALGON;
- les polymères de type poly(ammonium quaternaire) préparés et décrits dans le brevet français 2 270 846, constitués de motifs récurrents répondant à la formule (XXI) suivante 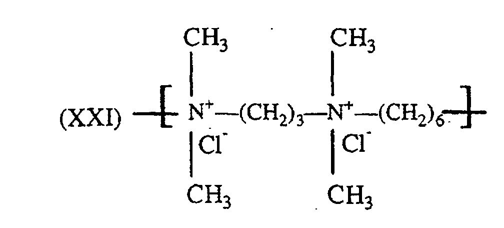 et notamment ceux dont la masse molaire moyenne en poids, déterminée par chromatographie par perméation de gel, est comprise entre 9500 et 9900 ;
- les polymères de type poly(ammonium quaternaire) préparés et décrits dans le brevet français 2 270 846, constitués de motifs récurrents répondant à la formule (XXII) suivante 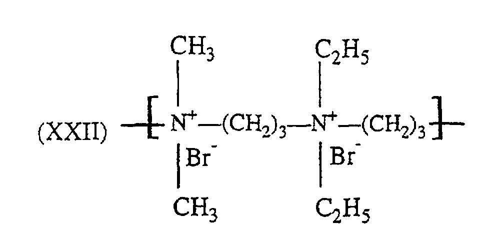 et notamment ceux dont la masse molaire moyenne en poids, déterminée par chromatographie par perméation de gel, est d'environ 1200 ;
- les polymères de type poly(ammonium quaternaire) décrits dans les brevets US 4 390 689, 4 702 906, 4 719 282 et constitués de motifs récurrents répondant à la formule (XXIII) suivante : 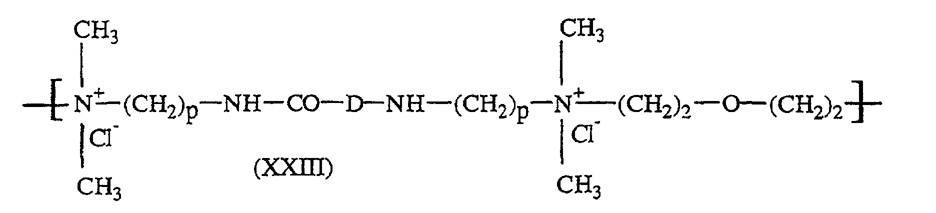 dans laquelle
   p est un nombre entier valant entre 1 et 6,
   D représente une simple liaison ou un groupe -(CH₂)ᵣ-CO- où r vaut 4 ou 7,
      et notamment ceux dont la masse molaire moyenne en poids est inférieure à 100 000, de préférence inférieure ou égale à 50 000;
   - les copolymères amphotères suivants :
      - le copolymère chlorure de diallyldiméthylammonium/acide acrylique (80/20) commercialisé sous la dénomination MERQUAT® 280 DRY par la société CALGON (dénomination CTFA : Polyquaternium-22);
      - le copolymère chlorure de diméthyldiallylammonium/acide acrylique (95/5) vendu sous la dénomination MERQUAT® 295 DRY par la société CALGON (dénomination CTFA : Polyquaternium-22);
      - le copolymère de chlorure de méthacrylamidopropyltrimonium, d'acide acrylique et d'acrylate de méthyle, commercialisé sou la dénomination MERQUAT® 2001 par la société CALGON (dénomination CTFA : Polyquaternium-47) ; et
      - le terpolymère acrylamide/chlorure de diméthyldiallylammonium/acide acrylique commercialisé sous la dénomination MERQUAT® PLUS 3330 DRY par la société CALGON (dénomination CTFA : Polyquaternium-39).

Dans la liste des polymères substantifs ci-dessus, on préfère tout particulièrement les copolymères amphotères Polyquaternium-22, Polyquaternium-39 et Polyquaternium-47 (dénominations CTFA).

Les quantités de polymères substantifs cationiques ou amphotères dans les compositions selon la présente invention sont généralement comprises entre 0,03 et 30 % en poids rapporté au poids total de la composition.

Les polymères épaississants hydrosolubles utilisables selon la présente invention englobent tous les polymères hydrosolubles synthétiques ou d'origine naturelle utilisés classiquement dans le domaine cosmétique.

On peut citer comme exemples de polymères épaississants synthétiques la polyvinylpyrrolidone, l'acide polyacrylique, le polyacrylamide, l'acide polyacrylamidométhylpropanesulfonique ou des copolymères de ceux-ci, ces polymères étant réticulés ou non réticulés.

Les polymères épaississants d'origine naturelle utilisables selon la présente invention sont des polymères comportant au moins un motif de sucre, à savoir
(a) les gommes de guar non-ioniques ;
(b) les gommes de biopolysaccharides d'origine microbienne telles que les gommes de scléroglucane et de xanthane ;
(c) les gommes issues d'exsudats végétaux telles que la gomme arabique, la gomme ghatti, la gomme karaya ou la gomme adragante ;
(d) les gommes extraites d'algues telles que les carraghénanes ou l'agar,
(e) les gommes issues d'extraits végétaux tels que la gomme de caroube ou les pectines extraites de pulpes de fruits ;
(e) les alginates ;
(f) les amidons ; et
(g) les hydroxyalkylcelluloses et carboxyalkylcelluloses ;

On entend par le terme "motif de sucre" dans la présente invention une portion monosaccharidique ou une portion oligo- ou polysaccharidique constituée d'un même type de motifs saccharidiques (oligo- ou poly*holo*sides) ou de plusieurs types de motifs saccharidiques différents (oligo- ou poly*hétéro*sides).

Les unités saccharidiques de tous ces polymères peuvent porter un ou plusieurs substituants, par exemple des groupements alkyle, hydroxyalkyle, alcoxy, acyloxy ou carboxyle, les radicaux alkyle comportant de 1 à 4 atomes de carbone.

Les gommes de guar non ioniques peuvent être modifiées ou non modifiées. Les gommes de guar non modifiées sont par exemple des produits vendus sous la dénomination VIDOGUM® GH 175 par la société UNIPECTINE et sous la dénomination JAGUAR® C par la société MAYHALL.

Selon la présente invention, on peut aussi utiliser des gommes de guar non ioniques modifiées par des groupements hydroxyalkyle en C₁₋ 4, par exemple hydroxyméthyle, hydroxyéthyle, hydroxypropyle et hydroxybutyle.

Ces gommes de guar modifiées sont bien connues dans la technique et peuvent être préparées par réaction de la gomme de guar avec les oxydes d'alkylène appropriés. Le taux d'hydroxyalkylation (rapport du nombre de molécules d'oxyde d'alkylène fixées au nombre initial de groupes hydroxyle libres) est de préférence compris entre 0,4 et 1,2.

De telles gommes de guar non ioniques modifiées sont par exemple vendues sous les dénominations JAGUAR® HP8, JAGUAR® HP60, JAGUAR® HP120, JAGUAR® DC293 et JAGUAR® HP 105 par la société RHONE POULENC (MAYHALL) ou sous la dénomination GALACTASOL® 4H4FD2 par la société AQUALON.

Les gommes de biopolysaccharides d'origine microbienne telles que les gommes de scléroglucane ou de xanthane, les gommes issues d'exsudats végétaux telles que la gomme arabique, la gomme ghatti, la gomme karaya, la gomme adragante, les extraits d'algues tels que les carraghénanes ou l'agar, les extraits végétaux tels que la gomme de caroube ou les pectines, les alginates, les amidons, les hydroxyalkylcelluloses et carboxyalkylcelluloses sont bien connus de l'homme de métier et sont décrits notamment dans "Handbook of Water Soluble Gums and Resins" de Robert L. Davidson, édité chez Mc Graw Hill Book Company(1980).

Parmi ces gommes, les scléroglucanes sont représentés par les produits vendus par la société SANOFI BIO INDUSTRIES sous la dénomination ACTIGUM® CS, et en particulier sous la dénomination ACTIGUM® CS 11, et par la société ALBAN MULLER INTERNATIONAL sous la dénomination AMIGEL® .

On peut également utiliser d'autres scléroglucanes, par exemple un scléroglucane traité au glyoxal décrit dans la demande de brevet FR-A-2 633 940.

Les gommes de xanthane utilisables en tant qu'épaississants dans les compositions de la présente invention sont par exemple représentées par les produits vendus sous les dénominations KELTROL® , KELTROL T, KELTROL® TF, KELTROL® BT, KELTROL® RD et KELTROL® CG par la société NUTRASWEET KELCO, ou sous les dénominations RHODICARE® S ou RHODICARE® H par la société RHODIA CHIMIE.

Les hydroxyalkylcelluloses sont généralement des hydroxy(alkyl en C₁₋₄)-celluloses et plus particulièrement des hydroxyéthylcelluloses. Elles sont disponibles par exemple sous les dénominations CELLOSIZE® QP3L, CELLOSIZE® QP4400H, CELLOSIZE® QP30000H, CELLOSIZE® HEC30000A ou CELLOSIZE POLYMER® PCG10 par la société AMERCHOL, sous les dénominations NATROSOL® 250HHR, NATROSOL® 250MR, NATROSOL® 250M, NATROSOL® 250HHXR, NATROSOL® 250HHX, NATROSOL® 250HR, NATROSOL® HX par la société HERCULES ou encore sous la dénomination TYLOSE® H1000 par la société HOECHST.

Les hydroxyalkylcelluloses peuvent également être des hydroxypropylcelluloses vendues sous les dénominations KLUCEL® EF, KLUCEL® H, KLUCEL® LHF, KLUCEL® MF, KLUCEL® G par la société AQUALON.

Parmi les carboxyalkylcelluloses, on utilise de préférence la carboxyméthylcellulose qui est commercialisée par exemple sous les dénominations BLANOSE® 7M8/SF, BLANOSE® RAFFINÉE 7M, BLANOSE® 7LF, BLANOSE® 7MF, BLANOSE® 9M31F, BLANOSE® 12M31XP, BLANOSE® 12M31P, BLANOSE® 9M31XF, BLANOSE® 7H, BLANOSE® 7M31, BLANOSE® 7H3SXF par la société AQUALON, ou sous les dénominations AQUASORB® A500 et AMBERGUM® 1221 par la société HERCULES, sous les dénominations CELLOGEN® HP810A et CELLOGEN® HP6HS9 par la société MONTELLO, ou encore sous la dénomination PRIMELLOSE® par la société AVEBE.

Les polymères épaississants hydrosolubles particulièrement préférés utilisables en tant qu'agents épaississants classiques dans la composition décolorante anhydre de la présente invention sont les gommes de guar, les dérivés de gomme de guar ou les hydroxyalkylcelluloses.

Le ou les épaississant(s) hydrosoluble(s) décrit(s) ci-dessus sont utilisés généralement à raison de 0,03 à 30 % en poids, de préférence à raison de 0,3 à 15 % en poids, par rapport à la composition anhydre.

La présente invention à également pour objet un procédé de décoloration des fibres kératiniques, en particulier des cheveux humains.

Ce procédé comprend les étapes consistant
- à préparer la composition prête à l'emploi décrite ci-dessus,
- à appliquer ladite composition sur la zone des fibres kératiniques à décolorer,
- à laisser reposer pendant un temps suffisant pour obtenir la décoloration recherchée, temps généralement compris entre 10 minutes et une heure, de préférence entre 10 et 45 minutes, et
- à éliminer le mélange de décoloration par rinçage à l'eau, suivi d'un lavage avec un shampooing, puis d'un séchage.

Un autre objet de l'invention est un dispositif d'emballage en plusieurs parties, également appelé "kit" d'emballage, comprenant au moins trois compartiments dont le premier contient un sel peroxygéné tel que défini ci-dessus, le second contient un solvant hydrosoluble tel que défini ci-dessus et l'agent alcalin et le troisième contient une composition aqueuse de peroxyde d'hydrogène, le ou les polymère(s) amphiphile(s) non ionique(s) et/ ou anionique(s) comportant au moins une chaîne grasse étant introduit(s) dans un ou plusieurs de ces compartiments.

L'exemple, donné ci-après à titre purement illustratif et non limitatif, permet de mieux comprendre l'invention.

### Exemple 1

| **Composition A** | |
|---|---|
| alcool décylique éthoxylé avec 3 moles d'OE | 30 g |
| alcool décylique éthoxylé avec 5 moles d'OE | 26 g |
| acide oléique | 10 g |
| alcool oléique | 3 g |
| propylèneglycol | 9 g |
| alcool oléocétylique éthoxylé par 30 moles d'OE | 4,5 g |
| monobutyléther d'éthylèneglycol | 3 g |
| ammoniaque à 20,5 % en NH₃ | 9 g |
| eau | qsp 100 g |

| **Composition B** | |
|---|---|
| persulfate de sodium | 26 g |
| persulfate de potassium | 40 g |
| métasilicate de sodium | 14 g |
| chlorure d'ammonium | 5 g |
| EDTA | 1 g |
| laurylsulfate de sodium | 2 g |
| cétylhydroxyéthylcellulose | 2 g |
| silice | qsp 100 g |

On mélange 60 g de la composition A, 20 g de la composition B et 100 g de solution aqueuse de peroxyde d'hydrogène à 6 % en poids.

La viscosité du mélange varie très peu dans le temps et le mélange possède un bon pouvoir décolorant.

### Exemple 2

On a préparé la composition de décoloration suivante (quantités en % en poids)

| | |
|---|---|
| Acide laureth-5 carboxylique | 4 |
| Ethanolamine | 0,6 |
| Deceth-3 | 4 |
| Deceth-5 | 1,5 |
| PPG Myristyl éther | 2 |
| Oleth-10 | 1,5 |
| Alcool oléique | 1,5 |
| Ethanol | 5 |
| Méthoxyisopropanol | 6,5 |
| Polyglyceryl-4 oleyl éther | 2 |
| Merquat 100 | 1,5 |
| Serad FX 1100 | 1 |
| Peroxyde d'hydrogène | 3 |
| Séquestrant | 0,2 |
| Parfum | 1 |
| Eau | q.s.p 100 |

Cette composition destinée à être appliquée immédiatement sur cheveux à décolorer a été appliquée pendant 45 minutes sous casque.

Après rinçage et séchage, on a obtenu une décoloration homogène.

## Revendications

1. Composition aqueuse prête à l'emploi pour la décoloration de fibres kératiniques, en particulier de fibres kératiniques humaines, comprenant dans un milieu approprié pour la décoloration, au moins un agent alcalin, au moins un sel peroxygéné, du peroxyde d'hydrogène et au moins un solvant hydrosoluble, **caractérisée par le fait qu'**elle contient en outre au moins un polymère amphiphile non ionique et/ou anionique comportant au moins une chaîne grasse.

2. Composition selon la revendication 1, **caractérisée par le fait que** ledit polymère amphiphile non ionique comportant au moins une chaîne grasse est choisi parmi
- les celluloses ou hydroxyalkylcelluloses modifiées par des groupements comportant au moins une chaîne grasse de type alkyle, arylalkyle ou alkylaryle contenant un groupe alkyle en C₈₋₂₂, ou par des groupes alkylphénol polyalcoxylés ;
- les hydroxypropylguars modifiés par des groupements comportant au moins une chaîne grasse en C₈₋₂₂ ;
- les polyuréthannes comportant au moins une chaîne grasse de type alkyle ou alcényle en C₈₋₃₀ ;
- les copolymères de vinylpyrrolidone et de monomères hydrophobes à chaîne grasse,
- les copolymères de (méth)acrylates d'alkyle en C₁₋₆ et de monomères amphiphiles comportant au moins une chaîne grasse,
- les copolymères de (méth)acrylates hydrophiles et de monomères hydrophobes comportant au moins une chaîne grasse.

3. Composition selon la revendication 1 ou 2, **caractérisée par le fait que** le polymère amphiphile non ionique est une hydroxyéthylcellulose modifiée par des groupements comportant au moins un radical alkyle en C₈₋₂₂ ou un polyuréthanne comportant au moins une chaîne alkyle en C₁₀₋₂₀.

4. Composition selon la revendication 1, **caractérisée par le fait que** le polymère amphiphile anionique comportant au moins une chaîne grasse est un copolymère comprenant
- des motifs hydrophiles dérivés d'un ou de plusieurs monomères à insaturation éthylénique portant une fonction d'acide carboxylique, et
- des motifs hydrophobes dérivés d'un ou de plusieurs monomères à insaturation éthylénique portant une chaîne latérale hydrophobe.

5. Composition selon la revendication 4, **caractérisée par le fait que** le ou les monomères à insaturation éthylénique portant une fonction d'acide carboxylique sont choisis parmi l'acide éthacrylique, l'acide méthacrylique et l'acide acrylique, de préférence parmi l'acide méthacrylique, l'acide acrylique et des mélanges de ceux-ci.

6. Composition selon l'une des revendications 4 et 5, **caractérisé par le fait que** le ou les monomères à insaturation éthylénique portant une chaîne latérale hydrophobe sont choisis parmi les éthacrylates, méthacrylates et/ou acrylates d'alkyle en C₁₀₋₃₀, de préférence en C₁₂₋₂₂.

7. Composition selon l'une quelconque des revendications 4 à 6, **caractérisée par le fait que** le ou les monomères à insaturation éthylénique portant une chaîne latérale hydrophobe sont choisis parmi les éthers allyliques d'alcools gras correspondant à la formule
(I) CH₂=CR'CH₂-O-Bₙ-R
dans laquelle
R' représente un atome d'hydrogène ou un groupe méthyle,
B représente un groupe éthylèneoxy,
n est un nombre entier valant entre 0 et 100,
R représente un groupe hydrocarboné choisi parmi les restes alkyle, arylalkyle, aryle, alkylaryle ou cycloalkyle comportant de 8 à 30 atomes de carbone, de préférence de 10 à 24 atomes de carbone.

8. Composition selon l'une quelconque des revendications 4 à 7, **caractérisée par le fait que** le polymère amphiphile anionique comportant au moins une chaîne grasse comprend en outre des motifs dérivés d'un monomère réticulant contenant deux doubles liaisons éthyléniques non conjuguées.

9. Composition selon la revendication 8, **caractérisée par le fait que** ledit monomère réticulant est choisi parmi le phtalate de diallyle, le (méth)acrylate d'allyle, le divinylbenzène, le diméthacrylate de (poly)éthylèneglycol, le méthylène-bis-acrylamide, le polyallylsucrose ou le polyallylpentaérythritol.

10. Composition selon l'une quelconque des revendications 4 à 9, **caractérisée par le fait que** le polymère amphiphile anionique comportant au moins une chaîne grasse est un copolymère réticulé d'acide acrylique et d'acrylate d'alkyle en C₁₀₋₃₀.

11. Composition selon l'une quelconque des revendications précédentes, **caractérisée par le fait que** les polymères amphiphiles non ioniques et/ou anioniques comportant au moins une chaîne grasse sont présents à raison de 0,01 à 10 % en poids, de préférence à raison de 0,1 à 5 % en poids, par rapport au poids total de la composition aqueuse.

12. Composition selon l'une quelconque des revendications précédentes, **caractérisée par le fait que** le solvant hydroluble est choisi parmi les monoalcools aliphatiques linéaires ou ramifiés en C₁₋₆, les polyols aliphatiques linéaires ou ramifiés en C₃₋₂₀, les éthers de polyols dont les mono- ou di-éthers aliphatiques en C₁₋₆ de polyols en C₂₋₉ et les éthers aromatiques en C₆₋₉ de polyols en C₂₋₉.

13. Composition selon l'une quelconque des revendications précédentes, **caractérisée par le fait qu'**elle contient de 0,1 à 10 % en poids de solvant hydrosoluble, et de préférence de 0,5 à 8 % en poids de solvant hydrosoluble, par rapport au poids total de la composition aqueuse.

14. Composition selon l'une quelconque des revendications précédentes, **caractérisée par le fait que** l'agent alcalin est choisi parmi l'ammoniaque et les amines organiques telles que la mono-, di- ou triéthanolamine, le 2-amino-2-méthyl-1-propanol, le diaminopropane, les hydroxyalkylamines et les éthylènediamines oxyéthylénées et/ou oxypropylénées.

15. Composition selon l'une quelconque des revendications précédentes, **caractérisée par le fait que** le sel peroxygéné est choisi parmi les persulfates, percarbonates et perborates d'ammonium et de métaux alcalins, en particulier parmi les persulfates de sodium et de potassium.

16. Composition selon l'une quelconque des revendications précédentes, **caractérisée par le fait qu'**elle contient de 2 à 20 % en poids, et de préférence de 4 à 15 % en poids, de sel peroxygéné.

17. Composition selon l'une quelconque des revendications précédentes, **caractérisée par le fait qu'**elle contient de 0,5 % à 10 % en poids, et de préférence de 1 à 8 % en poids, de peroxyde d'hydrogène.

18. Composition selon l'une quelconque des revendications précédentes, **caractérisée par le fait qu'**elle contient en outre des adjuvants de décoloration choisis parmi les agents de contrôle du dégagement d'oxygène, les agents tensioactifs anioniques, non ioniques, cationiques, amphotères ou zwitterioniques et leurs mélanges, les huiles minérales et végétales, les cires, les liants, les charges minérales, les opacifiants, les colorants, les séquestrants, les parfums et les polymères.

19. Composition selon la revendication 18, **caractérisée par le fait qu'**elle contient au moins un polymère épaississant hydrosoluble naturel et/ou synthétique.

20. Composition selon la revendication 19, **caractérisée par le fait qu'**elle contient de 0,03 à 30 % en poids, de préférence de 0,3 à 15 % en poids d'au moins un polymère épaississant hydrosoluble.

21. Composition selon la revendication 18, **caractérisée par le fait qu'**elle contient au moins un polymère substantif cationique et/ou amphotère.

22. Composition selon la revendication 21, **caractérisée par le fait qu'**elle contient de 0,03 à 30 % en poids d'au moins un polymère substantif cationique et/ou amphotère.

23. Composition selon la revendication 18, **caractérisée par le fait qu'**elle contient de 0,01 à 40 % en poids et de préférence de 0,1 à 30 % en poids d'au moins un agent tensioactif.

24. Procédé de décoloration de fibres kératiniques, en particulier de cheveux humains, comprenant les étapes consistant
- à préparer, immédiatement avant l'emploi, une composition décolorante aqueuse définie selon l'une quelconque des revendications 1 à 23,
- à appliquer ladite composition sur la zone des fibres kératiniques à décolorer,
- à laisser reposer pendant un temps suffisant pour obtenir la décoloration recherchée, et
- à éliminer le mélange décolorant par rinçage à l'eau suivi d'un lavage avec un shampooing, puis d'un séchage.

25. Dispositif à plusieurs compartiments, ou "kit", pour la décoloration des fibres kératiniques et en particulier des fibres kératiniques humaines telles que les cheveux, **caractérisé par le fait qu'**il comporte au moins trois compartiments dont le premier contient un sel peroxygéné tel que défini dans la revendication 15, le second contient un solvant hydrosoluble tel que défini dans la revendication 12 et un agent alcalin tel que défini dans la revendication 14 et le troisième contient une composition aqueuse de peroxyde d'hydrogène, le ou les polymère(s) amphiphile(s) non ionique(s) et / ou anionique(s) comportant au moins une chaîne grasse étant introduit(s) dans un ou plusieurs de ces compartiments.

## Patentansprüche

1. Gebrauchsfertige wäßrige Zusammensetzung zum Bleichen von Keratinfasern und insbesondere menschlichen Keratinfasern, die in einem zum Bleichen geeigneten Medium mindestens eine Base, mindestens ein Peroxidsalz, Wasserstoffperoxid und mindestens ein wasserlösliches Lösungsmittel enthält, **dadurch gekennzeichnet, daß** ferner mindestens ein nichtionisches und/oder anionisches amphiphiles Polymer, das mindestens eine Fettkette aufweist, enthält.

2. Zusammensetzung nach Anspruch 1, **dadurch gekennzeichnet, daß** das nichtionische amphiphile Polymer, das mindestens eine Fettkette aufweist, ausgewählt ist unter:
- Cellulosen oder Hydroxyalkylcellulosen, die mit Gruppen, die mindestens eine Fettkette vom Typ einer Alkyl-, Arylalkyl- oder Alkylarylgruppe aufweisen, die eine C₈₋₂₂-Alkylgruppe enthalten, oder mit polyalkoxylierten Alkylphenolgruppen modifiziert sind;
- Hydroxypropylguarverbindungen, die mit Gruppen modifiziert sind, die mindestens eine C₈₋₂₂-Fettkette aufweisen;
- Polyurethanen, die mindestens eine Fettkette vom Alkyltyp oder Alkenyltyp mit 8 bis 30 Kohlenstoffatomen aufweisen;
- Copolymeren von Vinylpyrrolidon und hydrophoben Monomeren mit Fettkette;
- Copolymeren von C₁₋₆-Alkyl(meth)acrylaten und amphiphilen Monomeren, die mindestens eine Fettkette aufweisen; und
- Copolymeren von hydrophilen (Meth)acrylaten und hydrophoben Monomeren, die mindestens eine Fettkette aufweisen.

3. Zusammensetzung nach Anspruch 1 oder 2, **dadurch gekennzeichnet, daß** das nichtionische amphiphile Polymer eine Hydroxyethylcellulose, die mit Gruppen modifiziert ist, die mindestens eine C₈₋₂₂-Alkylgruppe enthalten, oder ein Polyurethan ist, das mindestens eine C₁₀₋₂₀-Alkylgruppe aufweist.

4. Zusammensetzung nach Anspruch 1, **dadurch gekennzeichnet, daß** das anionische amphiphile Polymer, das mindestens eine Fettkette aufweist, ein Copolymer ist, das enthält:
- *hydrophile* Einheiten, die von einem oder mehreren Monomeren mit ethylenischer Doppelbindung abgeleitet sind, die eine Carbonsäuregruppe aufweisen, und
- *hydrophobe* Einheiten, die von einem oder mehreren Monomeren mit ethylenischer Doppelbindung abgeleitet sind, die eine hydrophobe Seitenkette aufweisen.

5. Zusammensetzung nach Anspruch 4, **dadurch gekennzeichnet, daß** das Monomer oder die Monomere mit ethylenischer Doppelbindung, die eine Carboxygruppe tragen, unter Ethacrylsäure, Methacrylsäure und Acrylsäure und vorzugsweise Methacrylsäure und Acrylsäure und ihren Gemischen ausgewählt sind.

6. Zusammensetzung nach einem der Ansprüche 4 und 5, **dadurch gekennzeichnet, daß** das Monomer oder die Monomere mit ethylenischer Doppelbindung, die eine hydrophobe Seitenkette aufweisen, unter den Ethacrylaten, Methacrylaten und/oder Acrylaten von C₁₀₋₃₀-Alkyl und vorzugsweise C₁₂₋₂₂-Alkyl ausgewählt sind.

7. Zusammensetzung nach einem der Ansprüche 4 bis 6, **dadurch gekennzeichnet, daß** das Monomer- oder die Monomere mit ethylenischer Doppelbindung, die eine hydrophobe Seitenkette aufweisen, unter den Fettalkoholallylethem der folgenden Formel ausgewählt sind:
(I) CH₂=CR'CH₂-O-Bₙ-R
worin bedeuten:
R' ein Wasserstoffatom oder die Methylgruppe,
B Ethylenoxy,
n Null oder eine ganze Zahl von 1 bis 100,
R eine Kohlenwasserstoffgruppe, die unter Alkyl, Arylalkyl, Aryl, A1kylaryl oder Cycloalkyl mit 8 bis 30 Kohlenstoffatomen und vorzugsweise 10 bis 24 Kohlenstoffatomen ausgewählt ist.

8. Zusammensetzung nach einem der Ansprüche 4 bis 7, **dadurch gekennzeichnet, daß** das anionische amphiphile Polymer, das mindestens eine Fettkette aufweist, ferner Einheiten enthält, die von einem vernetzenden Monomer abgeleitet sind, das zwei nicht konjugierte ethylenische Doppelbindungen enthält.

9. Zusammensetzung nach Anspruch 8, **dadurch gekennzeichnet, daß** das vernetzende Monomer unter Diallylphthalat, Allyl(meth)acrylat, Divinylbenzol, (Poly)ethylenglykoldimethacrylat, Methylen-bisacrylamid, Polyallylsaccharose und Polyallylpentaerythrit ausgewählt ist.

10. Zusammensetzung nach einem der Ansprüche 4 bis 9, **dadurch gekennzeichnet, daß** das anionische amphiphile Polymer, das mindestens eine Fettkette aufweist, ein vernetztes Copolymer von Acrylsäure und C₁₀₋₃₀-Alkylacrylat ist.

11. Zusammensetzung nach einem der vorhergehenden Ansprüche, **dadurch gekennzeichnet, daß** die anionischen und/oder nichtionischen amphiphilen Polymere, die mindestens eine Fettkette aufweisen, in einem Mengenanteil von 0,01 bis 10 Gew.-% und vorzugsweise 0,1 bis 5 Gew.-%, bezogen auf das Gesamtgewicht der wäßrigen Zusammensetzung, vorliegen.

12. Zusammensetzung nach einem der vorhergehenden Ansprüche, **dadurch gekennzeichnet, daß** das wasserlösliche Lösungsmittel unter den geradkettigen oder verzweigten aliphatischen C₁₋₆-Monoalkoholen, den geradkettigen oder verzweigten aliphatischen C₃₋₂₀-Potyolen und den Polyolethern ausgewählt sind, darunter den aliphatischen Mono- oder Diethern mit 1 bis 6 Kohlenstoffatomen von C₂₋₉-Polyolen und den aromatischen Ethern mit 6 bis 9 Kohlenstoffatomen von C₂₋₉-Polyolen.

13. Zusammensetzung nach einem der vorhergehenden Ansprüche, **dadurch gekennzeichnet, daß** sie 0,1 bis 10 Gew.-% und vorzugsweise 0,5 bis 8 Gew.-% wasserlösliches Lösungsmittel, bezogen auf das Gesamtgewicht der wäßrigen Zusammensetzung, enthält.

14. Zusammensetzung nach einem der vorhergehenden Ansprüche, **dadurch gekennzeichnet, daß** die Base unter Ammoniak und organischen Aminen, wie Mono-, Di- und Triethanolamin, 2-Amino-2-methyl-1-propanol, Diaminopropan, Hydroxyalkylaminen und Ethylendiaminen, die ethoxyliert und/oder propoxyliert sind, ausgewählt ist.

15. Zusammensetzung nach einem der vorhergehenden Ansprüche, **dadurch gekennzeichnet, daß** das Peroxidsalz unter den Persulfaten, Percarbonaten und Perboraten von Ammonium oder Alkalimetallen und insbesondere Natriumpersulfat und Kaliumpersulfat ausgewählt ist.

16. Zusammensetzung nach einem der vorhergehenden Ansprüche, **dadurch gekennzeichnet, daß** sie 2 bis 20 Gew.-% und vorzugsweise 4 bis 15 Gew.-% Peroxidsalz enthält.

17. Zusammensetzung nach einem der vorhergehenden Ansprüche, **dadurch gekennzeichnet, daß** sie 0,5 bis 10 Gew.-% und vorzugsweise 1 bis 8 Gew.-% Wasserstoffperoxid enthält.

18. Zusammensetzung nach einem der vorhergehenden Ansprüche, **dadurch gekennzeichnet, daß** sie ferner Zusatzstoffe zum Bleichen enthält, die unter den Mitteln zur Regulierung der Sauerstoffentwicklung, anionischen, nichtionischen, kationischen, amphoteren oder zwitterionischen grenzflächenaktiven Stoffen und ihren Gemischen, Mineralölen, pflanzlichen Ölen, Wachsen, Bindemitteln, anorganischen Füllstoffen, Trübungsmitteln, Farbmitteln, Maskierungsmitteln, Parfums und Polymeren ausgewählt sind.

19. Zusammensetzung nach Anspruch 18, **dadurch gekennzeichnet, daß** sie mindestens ein wasserlösliches natürliches oder synthetisches verdickendes Polymer enthält.

20. Zusammensetzung nach Anspruch 19, **dadurch gekennzeichnet, daß** sie 0,03 bis 30 Gew.-% und vorzugsweise 0,3 bis 15 Gew.-% mindestens eines wasserlöslichen verdickenden Polymers enthält.

21. Zusammensetzung nach Anspruch 18, **dadurch gekennzeichnet, daß** sie mindestens ein kationisches und/oder amphoteres substantives Polymer enthält.

22. Zusammensetzung nach Anspruch 21, **dadurch gekennzeichnet, daß** sie 0,03 bis 30 Gew.-% kationisches und/oder amphoteres substantives Polymer enthält.

23. Zusammensetzung nach Anspruch 18, **dadurch gekennzeichnet, daß** sie 0,01 bis 40 Gew.-% und vorzugsweise 0,1 bis 30 Gew.-% mindestens eines grenzflächenaktiven Stoffes enthält.

24. Verfahren zum Bleichen von Keratinfasern und insbesondere menschlichen Haaren mit den folgenden Schritten:
- Herstellen einer wäßrigen Zusammensetzung zum Bleichen nach einem der Ansprüche 1 bis 23 unmittelbar vor der Anwendung,
- Auftragen der Zusammensetzung auf die zu blondierenden Bereiche der Keratinfasern,
- Einwirkenlassen während einer Zeitspanne, die für die gewünschte Blondierung ausreichend ist, und
- Entfernen des Gemisches zum Bleichen durch Spülen mit Wässer und anschließender Haarwäsche, worauf getrocknet wird.

25. Vorrichtung mit mehreren Abteilungen oder "Kit" zum Bleichen von Keratinfasern und insbesondere menschlichen Keratinfasern, wie dem Haar, **dadurch gekennzeichnet, daß** sie mindestens drei Abteilungen aufweist, wobei eine Abteilung ein Peroxidsalz, wie das in Anspruch 15 definierte Peroxidsalz, die zweite Abteilung ein wasserlösliches Lösemittel, wie das in Anspruch 12 definierte Lösemittel, und eine Base, wie eine in Anspruch 14 definierte Base, und die dritte Abteilung eine wäßrige Wasserstoffperoxid-Zubereitung enthält, wobei das oder die nichtionische(n) und/oder anionische(n) amphiphile(n) Polymer(e), die mindestens eine Fettkette aufweisen, eine oder mehrere dieser Abteilungen gegeben wird (werden).

## Claims

1. Ready-to-use aqueous composition for bleaching keratin fibres, in particular human keratin fibres, comprising, in a medium which is suitable for bleaching, at least one alkaline agent, at least one peroxygenated salt, hydrogen peroxide and at least one water-soluble solvent, **characterized in that** it contains, in addition, at least one nonionic and/or anionic amphiphilic polymer comprising at least one fatty chain.

2. Composition according to Claim 1, **characterized in that** the said nonionic amphiphilic polymer comprising at least one fatty chain is chosen from
- celluloses or hydroxyalkylcelluloses modified with groups comprising at least one fatty chain of alkyl, arylalkyl or alkylaryl type containing a C₈-C₂₂ alkyl group, or with polyalkoxylated alkylphenol groups;
- hydroxypropyl guars modified with groups comprising at least one C₈-C₂₂ fatty chain;
- polyurethanes comprising at least one fatty chain of C₈-C₃₀ alkyl or alkenyl type;
- copolymers of vinylpyrrolidone and of hydrophobic monomers containing a fatty chain;
- copolymers of C₁-C₆ alkyl (meth)acrylates and of amphiphilic monomers comprising at least one fatty chain;
- copolymers of hydrophilic (meth)acrylates and of hydrophobic monomers comprising at least one fatty chain.

3. Composition according to Claim 1 or 2, **characterized in that** the nonionic amphiphilic polymer is a hydroxyethylcellulose modified with groups comprising at least one C₈-C₂₂ alkyl radical or a polyurethane comprising at least one C₁₀-C₂₀ alkyl chain.

4. Composition according to Claim 1, **characterized in that** the anionic amphiphilic polymer comprising at least one fatty chain is a copolymer comprising
- hydrophilic units derived from one or more monomers containing ethylenic unsaturation bearing a carboxylic acid function, and
- hydrophobic units derived from one or more monomers containing ethylenic unsaturation bearing a hydrophobic side chain.

5. Composition according to Claim 4, **characterized in that** the monomer(s) containing ethylenic unsaturation bearing a carboxylic acid function is(are) chosen from ethacrylic acid, methacrylic acid and acrylic acid, preferably from methacrylic acid and acrylic acid and mixtures thereof.

6. Composition according to either of Claims 4 and 5, **characterized in that** the monomer(s) containing ethylenic unsaturation bearing a hydrophobic side chain is (are) chosen from C₁₀-C₃₀, preferably C₁₂-C₂₂, alkyl ethacrylates, methacrylates and/or acrylates.

7. Composition according to any one of Claims 4 to 6, **characterized in that** the monomer (s) containing ethylenic unsaturation bearing a hydrophobic side chain is(are) chosen from allyl fatty alkyl ethers corresponding to the formula
(I) CH₂=CR'CH₂-O-Bₙ-R
in which
R' represents a hydrogen atom or a methyl group,
B represents an ethylenoxy group,
n is an integer between 0 and 100,
R represents a hydrocarbon-based group chosen from alkyl, arylalkyl, aryl, alkylaryl and cycloalkyl residues comprising from 8 to 30 carbon atoms, preferably from 10 to 24 carbon atoms.

8. Composition according to any one of Claims 4 to 7, **characterized in that** the anionic amphiphilic polymer comprising at least one fatty chain also comprises units derived from a crosslinking monomer containing two non-conjugated ethylenic double bonds.

9. Composition according to Claim 8, **characterized in that** the said crosslinking monomer is chosen from diallyl phthalate, allyl (meth)acrylate, divinylbenzene, (poly)ethylene glycol dimethacrylate, methylenebisacrylamide, polyallylsucrose or polyallylpentaerythritol.

10. Composition according to any one of Claims 4 to 9, **characterized in that** the anionic amphiphilic polymer comprising at least one fatty chain is a crosslinked copolymer of acrylic acid and of C₁₀₋₃₀ alkyl acrylate.

11. Composition according to any one of the preceding claims, **characterized in that** the nonionic and/or anionic amphiphilic polymers comprising at least one fatty chain are present in a proportion of from 0.01 to 10% by weight, preferably in a proportion of from 0.1 to 5% by weight, relative to the total weight of the aqueous composition.

12. Composition according to any one of the preceding claims, **characterized in that** the water-soluble solvent is chosen from linear or branched C₁₋₆ aliphatic monoalcohols, linear or branched C₃₋₂₀ aliphatic polyols, polyol ethers including C₁₋₆ aliphatic mono- or diethers of C₂₋₉ polyols and C₆₋₉ aromatic ethers of C₂₋₉ polyols.

13. Composition according to any one of the preceding claims, **characterized in that** it contains from 0.1 to 10% by weight of water-soluble solvent, and preferably from 0.5 to 8% by weight of water-soluble solvent, relative to the total weight of the aqueous composition.

14. Composition according to any one of the preceding claims, **characterized in that** the alkaline agent is chosen from aqueous ammonia, organic amines such as monoethanolamine, diethanolamine, triethanolamine, 2-amino-2-methyl-1-propanol, diaminopropane, hydroxyalkylamines and ethylenediamines which are oxyethylenated and/or oxypropylenated.

15. Composition according to any one of the preceding claims, **characterized in that** the peroxygenated salt is chosen from the ammonium and alkali metal persulphates, percarbonates and perborates, in particular from sodium persulphate and potassium persulphate.

16. Composition according to any one of the preceding claims, **characterized in that** it contains from 2 to 20% by weight, and preferably from 4 to 15% by weight, of peroxygenated salt.

17. Composition according to any one of the preceding claims, **characterized in that** it contains from 0.5% to 10% by weight, and preferably from 1 to 8% by weight, of hydrogen peroxide.

18. Composition according to any one of the preceding claims, **characterized in that** it also contains bleaching adjuvants chosen from agents for controlling the release of oxygen, anionic, nonionic, cationic, amphoteric or zwitterionic surfactants and mixtures thereof, mineral or plant oils, waxes, binders, mineral fillers, opacifiers, dyes, sequestering agents, fragrances and polymers.

19. Composition according to Claim 18, **characterized in that** it contains at least one natural and/or synthetic water-soluble thickening polymer.

20. Composition according to Claim 19, **characterized in that** it contains from 0.03 to 30% by weight, preferably from 0.3 to 15% by weight, of at least one water-soluble thickening polymer.

21. Composition according to Claim 18, **characterized in that** it contains at least one cationic and/or amphoteric substantive polymer.

22. Composition according to Claim 21, **characterized in that** it contains from 0.03 to 30% by weight of at least one cationic and/or amphoteric substantive polymer.

23. Composition according to Claim 18, **characterized in that** it contains from 0.01 to 40% by weight, and preferably from 0.1 to 30% by weight, of at least one surfactant.

24. Process for bleaching keratin fibres, in particular human hair, comprising the steps consisting
- in preparing, immediately before use, an aqueous bleaching composition defined according to any one of Claims 1 to 23,
- in applying the said composition to the region of keratin fibres to be bleached,
- in leaving the mixture to stand on the fibres for a period which is sufficient to obtain the desired bleaching effect, and
- in removing the bleaching mixture by rinsing with water, followed by washing with a shampoo, and then drying.

25. Multi-compartment device or "kit" for bleaching keratin fibres, and in particular human keratin fibres such as the hair, **characterized in that** it comprises at least three compartments, the first of which contains a peroxygenated salt as defined in Claim 15, the second of which contains a water-soluble solvent as defined in Claim 12 and an alkaline agent as defined in Claim 14, and the third of which contains an aqueous hydrogen peroxide composition, the nonionic and/or anionic amphiphilic polymer(s) comprising at least one fatty chain being introduced into one or more of these compartments.
